# EUROPEAN PATENT SPECIFICATION

(11) **EP 4 434 491 B1**
(45) Date of publication and mention of the grant of the patent: **02.12.2026**
(21) Application number: 22892178.9
(22) Date of filing: 27.12.2022
(51) Int. Cl.: A61F 2/24

(54) **SELF-EXPANDING BIOLOGICAL VALVE**
SELBSTEXPANDIERENDE BIOLOGISCHE KLAPPE
VALVE BIOLOGIQUE AUTO-EXPANSIBLE

(30) Priority: 15.11.2021 CN 202111350732
(43) Date of publication of application: 25.09.2024
(73) Proprietor: Nanjing Saint Medical Technology Co., Ltd., Nanjing, Jiangsu 210061 (CN)
(72) Inventor: MA, Chenming, Nanjing, Jiangsu 210061 (CN); YU, Faxin, Nanjing, Jiangsu 210061 (CN); LI, Jiaxian, Nanjing, Jiangsu 210061 (CN); QIN, Xiang, Nanjing, Jiangsu 210061 (CN); PAN, Shouxiang, Nanjing, Jiangsu 210061 (CN)
(74) Representative: Gille Hrabal Partnerschaftsgesellschaft mbB Patentanwälte
(86) International application number: PCT/CN2022/142335
(87) International publication number: WO 2023/083387

(56) References cited:
- CN-A- 101 011 298
- CN-A- 102 458 309
- CN-A- 107 735 051
- CN-A- 109 414 322
- US-A1- 2014 188 221
- US-A1- 2017 071 732

## Description

The present application claims priority to the prior application with the patent application No. 202111350732.X entitled "SELF-EXPANDING BIOLOGICAL VALVE" and filed with the China National Intellectual Property Administration on November 15, 2021.

### TECHNICAL FIELD

The present disclosure belongs to the field of medical devices, and particularly relates to a self-expanding biological valve which is composed of materials such as nickel-titanium memory alloy, biological tissues and the like. The valve is implanted by surgery and suitable for the replacement of diseased valves, which can be applied to aortic valve, pulmonary valve, mitral valve and tricuspid valve positions.

### BACKGROUND

Damage to the heart valve from various pathogenic factors or congenital developmental malformations results in anatomical and functional abnormalities of one or more valves, manifested as valvular stenosis and/or regurgitation. The mitral valve and aortic valve are mainly affected (the tricuspid valve and pulmonary valve are less affected), and the abnormalities are manifested as pathological types such as mitral stenosis (MS), mitral regurgitation (MR), aortic stenosis (AS), aortic regurgitation (AR) and combined valvular disease.

In the prior art, valve replacement by surgery is currently more prone to minimally invasive and small-incision surgery, so as to reduce patient c and speed up recovery. Especially, when the original valve calcification cannot be removed, the anatomical position is not well exposed, and the traditional operation method has the problem that it is difficult to suture the valve. Moreover, if problems such as paravalvular leak and the like are inspected and found during the operation and the valve needs to be replaced again, the original suture needs to be carefully removed, which not only increases the operation time, but also increases the incidence rate of complications after the suture is performed again because the valve annulus has been damaged by suturing and knotting.

In recent years, transcatheter valve implantation has been increasingly applied to the treatment of valvular diseases, but it is difficult to accurately position the valve during implantation, which easily causes the valve implantation position to be inaccurate; for elderly patients with severe calcification, the original calcification focus is not resected, so that the valve and the valve annulus are difficult to be completely fit, and the paravalvular leak is easy to occur; in addition, stent implantation is also prone to displacement conditions, which will also affect the long-term prognosis of patients. Therefore, a self-expanding biological valve that is simple to remove and has no mechanical damage to the valve annulus is desired. Meanwhile, the self-expanding biological valve has relatively low requirements for surgical operation, is safe to implant, facilitates the implementation of minimally invasive surgery, and is convenient for young cardiac surgeons to master. The self-expanding biological valve implantation technology is feasible and safe, which can significantly benefit hemodynamics, can also significantly improve clinical symptoms, and shortens blood flow occlusion time and extracorporeal circulation time, thereby being able to effectively reduce the mortality and the recurrence rate and having a wide range of indications and application prospects.

Application US 2014/0188221 A discloses a prosthetic heart valve. The heart valve disclosed in this prior art is configured to replace a native heart valve and has a support frame configured to be reshaped into an expanded form in order to receive and/or support an expandable prosthetic heart valve therein.

### SUMMARY

Therefore, an object of the present disclosure is to provide a self-expanding biological valve. According to a special design, the valve replacement can be carried out without complex suturing, the extracorporeal circulation time is reduced, the incidence rate of complications of important organs such as heart, brain, liver, kidney and the like after operation is reduced, and the potential damage, for example, to the aortic root caused by surgical operations such as suturing, knotting and the like is also avoided.

The present disclosure is implemented by the following technical schemes:
The present disclosure provides a self-expanding biological valve, which comprises a fixing stent, an inflow end support structure, an outflow end support structure and a silica gel suture ring, wherein the fixing stent comprises three stretching parts, which are positioned at a lower edge position of junctions of three valve leaflets of the fixing stent, respectively; the inflow end support structure and the outflow end support structure are arranged on the fixing stent, and in an unfolded state, the inflow end support structure and the outflow end support structure extend outwards relative to the fixing stent in a radial direction and are used for supporting the valve at a predetermined position and continuously applying an outward and/or downward force to surrounding tissues, so as to ensure reliable fixation of the valve; the silica gel suture ring is arranged at a valve annulus plane position on the fixing stent, and the valve is suitable to be sutured with the surrounding human tissues in a three-point positioning way by the silica gel suture ring, so as to ensure that the three valve leaflets in the valve are positioned at proper positions in the valve annulus plane.

According to the valve of the present disclosure, the inflow end support structure and the outflow end support structure are support buckles or coronas.

According to the valve of the present disclosure, the support buckle structure is composed of a nickel-titanium alloy material and provided with petal-shaped protruding parts extending outwards in the unfolded state; the corona is composed of a uniformly distributed network structure, wherein the network structure is composed of a nickel-titanium alloy material, and a skirt composed of a polymer material is wrapped outside the corona; in the unfolded state, the diameter of the corona is larger than that of the valve annulus, thereby preventing valve displacement.

According to the valve of the present disclosure, the stretching part has a structure with a cross-sectional area smaller than that of other parts of the fixing stent; when the valve is used, the stretching part is broken by a radial expansion force applied from the outside, and the diameter of the fixing stent can be increased and the fixing stent can be expanded outwards after the stretching part is broken.

According to the valve of the present disclosure, the stretching part is in a shape of a Chinese character "ji ( 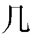 )", and the expected breaking position of the stretching part is positioned at a bending part thereof.

According to the valve of the present disclosure, the number of the protruding parts of the support buckle is 3 or more than 3, preferably a multiple of 3, and the protruding parts are uniformly distributed on the circumference of the fixing stent.

According to the valve of the present disclosure, the fixing stent is composed of a metal or polymer material.

According to the valve of the present disclosure, the inflow end support structure and the outflow end support structure are integrally formed.

According to the valve of the present disclosure, when the valve is used for a valve with a smaller diameter at the inflow end and a larger diameter at the outflow end, such as an aortic valve, the inflow end support structure is a corona, and the outflow end support structure is a support buckle.

According to the valve of the present disclosure, when the valve is used for a valve with a larger diameter at the inflow end and a smaller diameter at the outflow end, such as a tricuspid valve, the inflow end support structure is a support buckle, and the outflow end support structure is a corona.

According to the valve of the present disclosure, when the valve is used for a valve with smaller diameters at both the inflow end and the outflow end, such as a pulmonary valve, the inflow end support structure and the outflow end support structure are both coronas.

According to the valve of the present disclosure, when the valve is used for a valve with larger diameters at both the inflow end and the outflow end, such as a mitral valve, the inflow end support structure and the outflow end support structure are both support buckles.

### Beneficial Effects of Present Disclosure:

The present disclosure has advantages that the valve of the present disclosure can be used and subjected to valve replacement without complex suturing, the operation time is reduced, and the risk of complications after the operation is reduced; the ingenious design of the valve can not only achieve the rapid release of the valve stent, but also achieve reliable fixation of the valve stent. If problems such as paravalvular leak and the like are inspected and found during the operation and the valve needs to be replaced again, the self-expanding biological valve is simple to remove, has no mechanical damage to the valve annulus, has relatively low requirements for surgical operation, facilitates the implementation of minimally invasive surgery, and is convenient for young cardiac surgeons to master. The stretching breaking position of the stent is positioned at the position of the junction of the fixing stent valve leaflet and is completely covered by the polymer skirt. It is verified by experiments that the valve is very safe and reliable.

### BRIEF DESCRIPTION OF THE DRAWINGS

FIG. 1 is a schematic view of an embodiment of a self-expanding biological valve of the present disclosure for aortic valve replacement;
FIG. 2 is a partial enlarged view of a stretching part of a fixing stent of the self-expanding biological valve according to an embodiment of the present disclosure;
FIG. 3 is a schematic view of the self-expanding biological valve implanted in an aorta according to an embodiment of the present disclosure;
FIG. 4 is a top view of the self-expanding biological valve according to an embodiment of the present disclosure;
FIG. 5 is a schematic structural view of a single-layer fixing stent of the self-expanding biological valve according to an embodiment of the present disclosure;
FIG. 6 is a schematic structural view of a double-layer fixing stent of the self-expanding biological valve according to an embodiment of the present disclosure;
FIG. 7 is a schematic view of an outer-layer stent of the double-layer fixing stent of the self-expanding biological valve according to an embodiment of the present disclosure;
FIG. 8 is a schematic view of an inner-layer stent of the double-layer fixing stent of the self-expanding biological valve according to an embodiment of the present disclosure;
FIG. 9 shows a schematic view of an embodiment of the self-expanding biological valve of the present disclosure for pulmonary valve replacement;
FIG. 10 shows a schematic view of an embodiment of the self-expanding biological valve of the present disclosure for mitral valve replacement;
FIG. 11 shows a schematic view of an embodiment of the self-expanding biological valve of the present disclosure for tricuspid valve replacement.

### DETAILED DESCRIPTION

The present disclosure will be further illustrated with reference to the following specific examples. It should be understood that these examples are merely intended to illustrate the present disclosure rather than limit the protection scope of the present disclosure. In addition, it should be understood that various changes or modifications may be made by those skilled in the art after reading the teachings of the present disclosure.

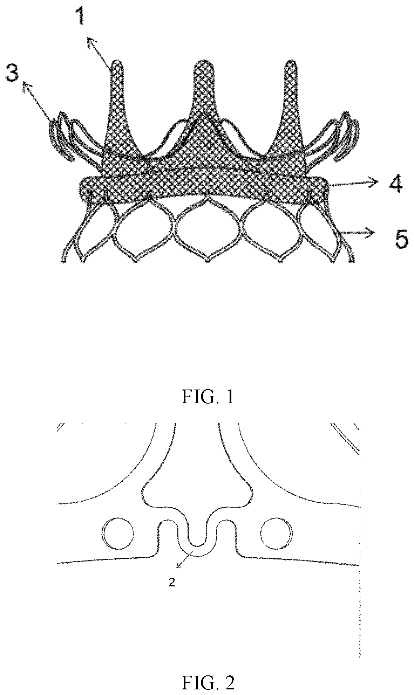

This embodiment takes a self-expanding biological valve for aortic valve replacement as an example, and the self-expanding biological valve of the present disclosure is specifically described as follows. As shown in FIG. 1, the self-expanding biological valve comprises a fixing stent 1, an outflow end support structure 3, a silica gel suture ring 4 and an inflow end support structure 5. The fixing stent 1 is composed of a metal or polymer material, such as PETG and ABS materials. The fixing stent 1 is provided with three stretching parts 2 (see FIG. 2), which are positioned at a lower edge position of junctions of three valve leaflets (not shown in the figure) of the fixing stent 1, respectively. The stretching part 2 is in a shape of a Chinese character " 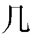 ". The cross-sectional area of the part is smaller than that of other parts of the fixing stent, so that the strength is relatively low, the part is easy to break when being subjected to a radially outward expansion force, and particularly, a bending part of the stretching part is easy to break.

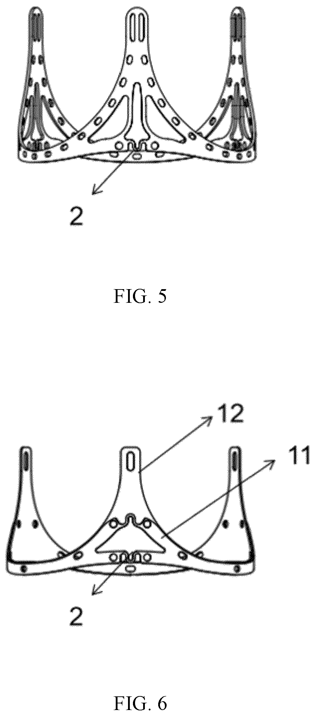

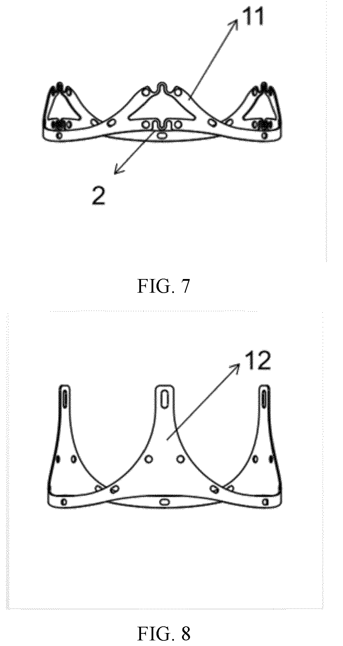

The fixing stent 1 can adopt various structures, and in this embodiment, two schemes of a single-layer stent structure and a double-layer stent structure are separately adopted. FIG. 5 shows the scheme of the single-layer stent structure; FIG. 6 shows the scheme of the double-layer stent structure. An outer-layer stent 11 is a short stent structure (see FIG. 7) composed of a metal material, an inner-layer stent 12 is a long stent structure (see FIG. 8) composed of a polymer material, and the two are fixed by suturing to form the fixing stent 1.

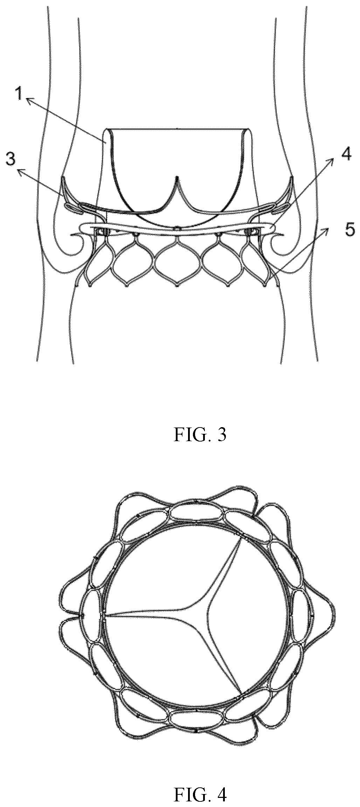

The outflow end support structure 3 adopts a support buckle structure, and the outflow end support structure 3 (i.e., the support buckle) is arranged on the fixing stent 1 and is positioned at an outflow end of the valve. In an unfolded state, the support buckle extends outwards relative to the fixing stent 1 in a radial direction (as shown in FIGs. 1 and 4) and is in an open petal shape for supporting the valve in the aortic sinus and continuously applying outward and downward forces to surrounding tissues, so as to ensure reliable fixation of the valve. The support buckle comprises three petal-shaped protruding parts, and the number of the protruding parts can also be more than three, preferably a multiple of 3. The protruding parts are uniformly distributed on the circumference of the fixing stent 1 and are all composed of nickel-titanium alloy materials.

The silica gel suture ring 4 is arranged at a valve annulus plane position on the fixing stent, and the artificial valve is sutured with the surrounding tissues in a three-point positioning way by the silica gel suture ring, thereby determining accurate positions of the three valve leaflets in the valve annulus plane. The inflow end support structure 5 adopts a corona structure, and a skirt composed of a polymer material is wrapped outside the corona (not shown in the figure). In the unfolded state, the diameter of the corona is larger than that of the valve annulus, and it applies an outward force to the surrounding tissues, thereby preventing valve displacement (as shown in FIG. 3). The corona 5 and the support buckle 3 are manufactured by adopting an integral forming and integral cutting mode. When the skirt is sutured, the corona 5 and the support buckle 3 are both connected to the fixing stent 1.

Before implantation, the valve is kept in a contracted state by tightening the support buckle 3 and the corona 5 through binding wires. When the valve is implanted in the aorta at a predetermined position, the support buckle 3 and the corona 5 are released naturally by removing the binding wires, and the support buckle 3 and the corona 5 can be released rapidly to a desired shape due to the excellent elasticity of the metal nickel-titanium alloy material. As shown in FIG. 3, the force of the support buckle 3 and the corona 5 on the tissues around the valve enables the valve to be fixed in the desired position and not easy to slide. Then, by extending an expanding device, such as a highpressure balloon device, into the valve stent and applying a radial expansion force to the valve stent, the stretching parts 2 on the fixing stent 1 are subjected to the expansion force to be broken, and the fixing stent 1 is rapidly expanded in the radial direction along with the breaking of each stretching part 2 to be tightly matched with the surrounding vascular tissues, thereby ensuring the reliable fixation of the valve.

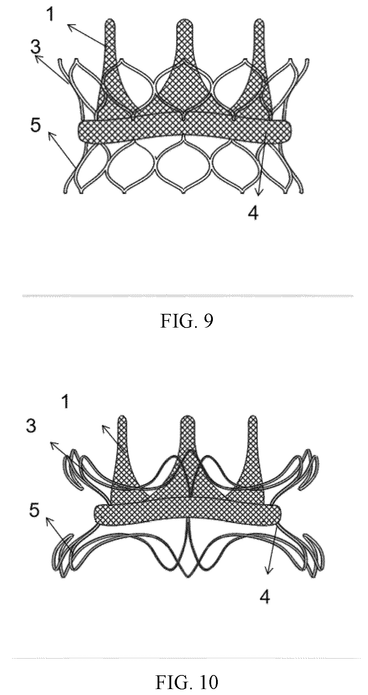

FIG. 9 shows an embodiment of the self-expanding biological valve of the present disclosure for pulmonary valve replacement. In this embodiment, the inflow end support structure 5 and the outflow end support structure 3 are both coronas due to smaller diameters of both the inflow end and the outflow end.

FIG. 10 shows an embodiment of the self-expanding biological valve of the present disclosure for mitral valve replacement. In this embodiment, the inflow end support structure 5 and the outflow end support structure 3 are both support buckles due to larger diameters of both the inflow end and the outflow end.

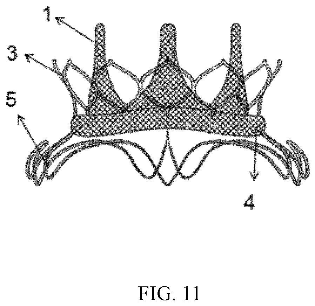

FIG. 11 shows an embodiment of the self-expanding biological valve of the present disclosure for tricuspid valve replacement. In this embodiment, the inflow end support structure 5 is a support buckle and the outflow end support structure 3 is a corona due to a larger diameter of the inflow end and a smaller diameter of the outflow end.

## Claims

1. A self-expanding biological valve, comprising a fixing stent (1), an inflow end support structure (5), an outflow end support structure (3) and a suture ring (4), wherein the fixing stent (1) comprises three stretching parts (2), wherein the inflow end support structure (5) and the outflow end support structure (3) are arranged on the fixing stent (1),
**characterized in that**,
the suture ring is a silica gel suture ring; the stretching parts (2) are positioned at a lower edge position of junctions of three valve leaflets of the fixing stent (1), respectively;, and in an unfolded state, the inflow end support structure (5) and the outflow end support structure (3) extend outwards relative to the fixing stent (1) in a radial direction and are used for supporting the valve at a predetermined position and continuously applying an outward and/or downward force to surrounding tissues, so as to ensure reliable fixation of the valve; the silica gel suture ring (4) is arranged at a valve annulus plane position on the fixing stent (1), and the valve is suitable to be sutured with the surrounding human tissues in a three-point positioning way by the silica gel suture ring (4), so as to ensure that the three valve leaflets in the valve are positioned at proper positions in the valve annulus plane.

2. The self-expanding biological valve according to claim 1, wherein the inflow end support structure (5) and the outflow end support structure (3) are support buckles or coronas.

3. The self-expanding biological valve according to claim 2, wherein the support buckle structure is composed of a nickel-titanium alloy material and provided with petal-shaped protruding parts extending outwards in the unfolded state; the corona is composed of a uniformly distributed network structure, wherein the network structure is composed of a nickel-titanium alloy material, and a skirt composed of a polymer material is wrapped outside the corona; in the unfolded state, the diameter of the corona is larger than that of the valve annulus, thereby preventing valve displacement.

4. The self-expanding biological valve according to any one of claims 1-3, wherein the stretching part (2) has a structure with a cross-sectional area smaller than that of other parts of the fixing stent (1); when the valve is used, the stretching part (2) is broken by a radial expansion force applied from the outside, and the diameter of the fixing stent (1) can be increased and the fixing stent (1) can be expanded outwards after the stretching part (2) is broken.

5. The self-expanding biological valve according to claim 4, wherein the stretching part (2) is in a shape of a Chinese character " 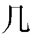 ", and the expected breaking position of the stretching part (2) is positioned at a bending part thereof.

6. The self-expanding biological valve according to any one of claims 3-5, wherein the number of the protruding parts of the support buckle is 3 or more than 3, preferably a multiple of 3, and the protruding parts are uniformly distributed on the circumference of the fixing stent (1).

7. The self-expanding biological valve according to any one of claims 1-6, wherein the fixing stent (1) is composed of a metal or polymer material.

8. The self-expanding biological valve according to any one of claims 1-7, wherein the inflow end support structure (5) and the outflow end support structure (3) are integrally formed.

9. The self-expanding biological valve according to any one of claims 2-8, wherein when the self-expanding biological valve is used for a valve with a smaller diameter at an inflow end and a larger diameter at an outflow end, the inflow end support structure (5) is a corona, and the outflow end support structure (3) is a support buckle.

10. The self-expanding biological valve according to any one of claims 2-8, wherein when the self-expanding biological valve is used for a valve with a larger diameter at an inflow end and a smaller diameter at an outflow end, the inflow end support structure (5) is a support buckle, and the outflow end support structure (3) is a corona.

11. The self-expanding biological valve according to any one of claims 2-8, wherein when the self-expanding biological valve is used for a valve with smaller diameters at both inflow end and outflow end, the inflow end support structure (5) and the outflow end support structure (3) are both coronas.

12. The self-expanding biological valve according to any one of claims 2-8, wherein when the self-expanding biological valve is used for a valve with larger diameters at both inflow end and outflow end, the inflow end support structure (5) and the outflow end support structure (3) are both support buckles.

## Patentansprüche

1. Selbstexpandierende biologische Klappe, umfassend einen Fixierungsstent (1), eine Einlassende-Stützstruktur (5), eine Auslassende-Stützstruktur (3) und einen Nahtring (4), wobei der Fixierungsstent (1) drei Dehnteile (2) umfasst, wobei die Einlassende-Stützstruktur (5) und die Auslassende-Stützstruktur (3) an dem Fixierungsstent (1) angeordnet sind,
**dadurch gekennzeichnet, dass**
der Nahtring ein Kieselgel-Nahtring ist; die Dehnteile (2) jeweils an einer unteren Randposition von Verbindungsstellen dreier Klappensegel des Fixierungsstents (1) positioniert sind; und sich in einem entfalteten Zustand die Einlassende-Stützstruktur (5) und die Auslassende-Stützstruktur (3) relativ zu dem Fixierungsstent (1) in einer radialen Richtung nach außen erstrecken und dazu verwendet werden, die Klappe an einer vorbestimmten Position zu stützen und kontinuierlich eine nach außen und/oder unten gerichtete Kraft auf umgebendes Gewebe auszuüben, um eine zuverlässige Fixierung der Klappe sicherzustellen; der Kieselgel-Nahtring (4) an einer Position in der Ebene eines Klappenannulus an dem Fixierungsstent (1) angeordnet ist und die Klappe dazu geeignet ist, mit dem umgebenden menschlichen Gewebe in einer Dreipunktpositionierungsweise durch den Kieselgel-Nahtring (4) vernäht zu werden, um sicherzustellen, dass die drei Klappensegel in der Klappe an richtigen Positionen in der Ebene des Klappenannulus positioniert sind.

2. Selbstexpandierende biologische Klappe nach Anspruch 1, wobei die Einlassende-Stützstruktur (5) und die Auslassende-Stützstruktur (3) Stützschnallen oder Kränze sind.

3. Selbstexpandierende biologische Klappe nach Anspruch 2, wobei die Stützschnallenstruktur aus einem Nickel-Titan-Legierungsmaterial gebildet ist und mit blütenblattförmigen vorstehenden Teilen versehen ist, die sich in dem entfalteten Zustand nach außen erstrecken; der Kranz aus einer gleichmäßig verteilten Netzstruktur gebildet ist, wobei die Netzstruktur aus einem Nickel-Titan-Legierungsmaterial gebildet ist und eine aus einem Polymermaterial gebildete Schürze außen um den Kranz gewickelt ist; und in dem entfalteten Zustand der Durchmesser des Kranzes größer als derjenige des Klappenannulus ist, wodurch eine Klappenverlagerung verhindert wird.

4. Selbstexpandierende biologische Klappe nach einem der Ansprüche 1 bis 3, wobei das Dehnteil (2) eine Struktur mit einer Querschnittsfläche aufweist, die kleiner als diejenige anderer Teile des Fixierungsstents (1) ist; wobei, wenn die Klappe verwendet wird, das Dehnteil (2) durch eine von außen ausgeübte radiale Expansionskraft gebrochen wird und der Durchmesser des Fixierungsstents (1) vergrößert werden kann und der Fixierungsstent (1) nach außen expandiert werden kann, nachdem das Dehnteil (2) gebrochen wurde.

5. Selbstexpandierende biologische Klappe nach Anspruch 4, wobei das Dehnteil (2) in Form eines chinesischen Schriftzeichens " 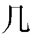 " ausgebildet ist und die erwartete Bruchposition des Dehnteils (2) an einem Biegeteil davon positioniert ist.

6. Selbstexpandierende biologische Klappe nach einem der Ansprüche 3 bis 5, wobei die Anzahl der vorstehenden Teile der Stützschnalle 3 oder mehr als 3, vorzugsweise ein Vielfaches von 3 beträgt und die vorstehenden Teile gleichmäßig über den Umfang des Fixierungsstents (1) verteilt sind.

7. Selbstexpandierende biologische Klappe nach einem der Ansprüche 1 bis 6, wobei der Fixierungsstent (1) aus einem Metall- oder Polymermaterial gebildet ist.

8. Selbstexpandierende biologische Klappe nach einem der Ansprüche 1 bis 7, wobei die Einlassende-Stützstruktur (5) und die Auslassende-Stützstruktur (3) einstückig ausgebildet sind.

9. Selbstexpandierende biologische Klappe nach einem der Ansprüche 2 bis 8, wobei, wenn die selbstexpandierende biologische Klappe als Klappe mit einem kleineren Durchmesser an einem Einlassende und einem größeren Durchmesser an einem Auslassende verwendet wird, die Einlassende-Stützstruktur (5) ein Kranz ist und die Auslassende-Stützstruktur (3) eine Stützschnalle ist.

10. Selbstexpandierende biologische Klappe nach einem der Ansprüche 2 bis 8, wobei, wenn die selbstexpandierende biologische Klappe als Klappe mit einem größeren Durchmesser an einem Einlassende und einem kleineren Durchmesser an einem Auslassende verwendet wird, die Einlassende-Stützstruktur (5) eine Stützschnalle ist und die Auslassende-Stützstruktur (3) ein Kranz ist.

11. Selbstexpandierende biologische Klappe nach einem der Ansprüche 2 bis 8, wobei, wenn die selbstexpandierende biologische Klappe als Klappe mit kleineren Durchmessern sowohl an einem Einlassende als auch an einem Auslassende verwendet wird, sowohl die Einlassende-Stützstruktur (5) als auch die Auslassende-Stützstruktur (3) Kränze sind.

12. Selbstexpandierende biologische Klappe nach einem der Ansprüche 2 bis 8, wobei, wenn die selbstexpandierende biologische Klappe als Klappe mit größeren Durchmessern sowohl an einem Einlassende als auch an einem Auslassende verwendet wird, sowohl die Einlassende-Stützstruktur (5) als auch die Auslassende-Stützstruktur (3) Stützschnallen sind.

## Revendications

1. Valve biologique auto-expansible, comprenant un stent de fixation (1), une structure de support d'extrémité d'entrée (5), une structure de support d'extrémité de sortie (3) et un anneau de suture (4), dans laquelle le stent de fixation (1) comprend trois parties extensibles (2), dans laquelle la structure de support d'extrémité d'entrée (5) et la structure de support d'extrémité de sortie (3) sont agencées sur le stent de fixation (1), **caractérisée en ce que**,
l'anneau de suture est un anneau de suture en gel de silice ; les parties extensibles (2) sont positionnées respectivement au niveau d'une position de bord inférieur de jonctions de trois feuillets valvulaires du stent de fixation (1) ; et à l'état déployé, la structure de support d'entrée (5) et la structure de support de sortie (3) s'étendent vers l'extérieur par rapport au stent de fixation (1) dans une direction radiale et servent à maintenir la valve au niveau d'une position prédéterminée et à appliquer en continu une force vers l'extérieur et/ou vers le bas sur les tissus environnants, afin de garantir une fixation fiable de la valve ; l'anneau de suture en gel de silice (4) est agencé au niveau d'un plan de l'anneau valvulaire sur le stent de fixation (1), et la valve est adaptée à être suturée aux tissus humains environnants selon un positionnement en trois points par l'anneau de suture en gel de silice (4), afin de garantir que les trois feuillets valvulaires dans la valve sont positionnés au niveau de positions appropriées dans le plan de l'anneau valvulaire.

2. Valve biologique auto-expansible selon la revendication 1, dans laquelle la structure de support d'extrémité d'entrée (5) et la structure de support d'extrémité de sortie (3) sont des boucles de support ou des couronnes.

3. Valve biologique auto-expansible selon la revendication 2, dans laquelle la structure de boucle de support est composée d'un alliage nickel-titane et munie de parties saillantes en forme de pétales s'étendant vers l'extérieur à l'état déployé ; la couronne est composée d'une structure en réseau uniformément répartie, dans laquelle la structure en réseau est composée d'un alliage nickel-titane, et une jupe composée d'un matériau polymère enveloppe la couronne ; à l'état déployé, le diamètre de la couronne est supérieur à celui de l'anneau valvulaire, empêchant ainsi le déplacement de la valve.

4. Valve biologique auto-expansible selon l'une quelconque des revendications 1 à 3, dans laquelle la partie extensible (2) a une structure avec une aire en coupe transversale plus petite que celle d'autres parties du stent de fixation (1) ; lorsque la valve est utilisée, la partie extensible (2) est rompue par une force d'expansion radiale appliquée de l'extérieur, et le diamètre du stent de fixation (1) peut être augmenté et le stent de fixation (1) peut être expansé vers l'extérieur après la rupture de la partie extensible (2).

5. Valve biologique auto-expansible selon la revendication 4, dans laquelle la partie extensible (2) a la forme d'un caractère chinois « 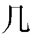 », et la position de rupture prévue de la partie extensible (2) est positionnée au niveau d'une partie courbée de celle-ci.

6. Valve biologique auto-expansible selon l'une quelconque des revendications 3 à 5, dans laquelle le nombre de parties saillantes de la boucle de support est de 3 ou plus de 3, de préférence un multiple de 3, et les parties saillantes sont uniformément réparties sur la circonférence du stent de fixation (1).

7. Valve biologique auto-expansible selon l'une quelconque des revendications 1 à 6, dans laquelle le stent de fixation (1) est composé d'un matériau métallique ou polymère.

8. Valve biologique auto-expansible selon l'une quelconque des revendications 1 à 7, dans laquelle la structure de support d'extrémité d'entrée (5) et la structure de support d'extrémité de sortie (3) sont formées d'un seul tenant.

9. Valve biologique auto-expansible selon l'une quelconque des revendications 2 à 8, dans laquelle, lorsque la valve biologique auto-expansible est utilisée pour une valve ayant un diamètre plus petit au niveau d'une extrémité d'entrée et un diamètre plus grand au niveau d'une extrémité de sortie, la structure de support d'extrémité d'entrée (5) est une couronne et la structure de support d'extrémité de sortie (3) est une boucle de support.

10. Valve biologique auto-expansible selon l'une quelconque des revendications 2 à 8, dans laquelle, lorsque la valve biologique auto-expansible est utilisée pour une valve ayant un diamètre plus grand au niveau d'une extrémité d'entrée et un diamètre plus petit au niveau d'une extrémité de sortie, la structure de support d'extrémité d'entrée (5) est une boucle de support et la structure de support d'extrémité de sortie (3) est une couronne.

11. Valve biologique auto-expansible selon l'une quelconque des revendications 2 à 8, dans laquelle, lorsque la valve biologique auto-expansible est utilisée pour une valve ayant des diamètres plus petits à la fois au niveau de l'extrémité d'entrée et de l'extrémité de sortie, la structure de support d'extrémité d'entrée (5) et la structure de support d'extrémité de sortie (3) sont toutes deux des couronnes.

12. Valve biologique auto-expansible selon l'une quelconque des revendications 2 à 8, dans laquelle, lorsque la valve biologique auto-expansible est utilisée pour une valve ayant des diamètres plus grands à la fois au niveau de l'extrémité d'entrée et de l'extrémité de sortie, la structure de support d'extrémité d'entrée (5) et la structure de support d'extrémité de sortie (3) sont toutes deux des boucles de support.
